# EUROPEAN PATENT APPLICATION

(11) **EP 4 549 888 A1**
(43) Date of publication of application: **07.05.2025**
(21) Application number: 23315407.9
(22) Date of filing: 31.10.2023
(51) Int. Cl.: G01G 19/50, A61B 5/0537

(54) **A WEIGHING SYSTEM WITH A PLURALITY OF MEASUREMENT SEQUENCES**

(71) Applicant: Withings, 92130 Issy-Les-Moulineaux (FR)
(72) Inventor: Carreel, Eric, 92130 Issy-les-Moulineaux (FR); Menanteau, Matthieu, 92130 Issy-les-Moulineaux (FR); Joussain, Antoine, 92130 Issy-les-Moulineaux (FR)
(74) Representative: Withings IP

(57) **Abstract**

A measurement system comprises a sensor device configured to generate configuration data during a configuration period and measurement data during a measurement period, the measurement data being representative of a physiological state of the user, and control circuitry configured to select a measurement sequence among a plurality of available measurement sequences, wherein each measurement sequence includes at least one physiological measurement of the user to generate measurement data, the selection being based at least on the configuration data.

The selection is based at least on an identification in the configuration data of a pattern amongst a plurality of predetermined patterns associated to the plurality of available measurement sequences.

The sensor device is further configured to generate the measurement data according to the selected measurement sequence.

## Description

### Field of the invention

The present invention relates to a weighing system. The weighing system is in particular a so called "bathroom scale" or "personal scale". Such a scale is notably configured to measure the weight of a user standing on the weighing system.

### Background

In addition to measuring weight, the latest up to date body scales are configured to measure more and more different physiological data to provide more health insights to the user on his/her body composition, his/her cardiovascular health, his/her nerve health, etc. For example, with a single step on the scale, the scale Withings Body Scan^{™} is able to provide a complete health scan by measuring a weight, a body composition, an electrocardiogram (ECG), a heart rate (HR), a Pulse Wave Velocity (PWV) and an electrochemical skin conductance (ESR) analysis.

However, it has been noted that for some users, this complete health check may take a bit too long, notably for users weighing themselves every day. As a consequence, these users tend to weigh themselves less frequently or to disactivate some of the measurements to reduce the length of the health check. This leads to a less effective health monitoring of these users which need to be address.

### Summary of the disclosure

An aim of the disclosure is to provide a weighing system enabling a more sophisticated approach with respect to the different available measurements for the user at each weighing session.

To that end, the disclosure relates to a measurement system comprising: a sensor device configured to generate configuration data during a configuration period and measurement data during a measurement period, the measurement data being representative of a physiological state of the user, control circuitry configured to select a measurement sequence among a plurality of available measurement sequences, wherein each measurement sequence includes at least one physiological measurement of the user to generate measurement data, the selection being based at least on the configuration data, wherein: the selection is based at least on an identification in the configuration data of a pattern amongst a plurality of predetermined patterns associated to the plurality of available measurement sequences, the sensor device is further configured to generate the measurement data according to the selected measurement sequence.

Thanks to this weighing system, the sensor device generates configuration data used by the control circuitry to selects a measurement sequence. The sensor device then generates measurement data according to the selected measurement sequence. By identifying for example a load pattern such as getting on the scale with alternatively the left or the right foot, which may be performed by the user each time he/she steps on the scale, the weighting system may switch the measurement sequence from an exhaustive health scan comprising several physiological measurements to a single measurement of the weight of the user. The user may therefore choose each time he/she steps on the scales which level of health insights he/she desires and may therefore adapt the duration of the measurement sequences. The user may for example require a complete health scan once a week and may require a faster measurement sequence the rest of the days of the week.

In an embodiment, each physiological measurement is associated to a physiological sign and at least a measurement parameter, said physiological measurement comprising the measurement of the physiological sign according to the at least one measurement parameter.

In an embodiment, the measurement parameter is a measurement duration, a choice of measurement algorithm, a choice of measurement sensor, etc

In an embodiment, the at least one physiological measurement comprises at least one of: weight or mass measurement, electrocardiogram measurement, impedance measurement including impedance-plethysmogram measurement, impedance-cardiogram measurement and/or bioimpedance analysis measurement, photoplethysmogram measurement, ballistocardiogram measurement, electrochemical skin conductance analysis ("ESC analysis") measurement, heart rate ("HR") measurement, pulse wave velocity ("PWV") measurement, and/or heart failure measurement.

In an embodiment, all the available measurement sequences are different from one another.

In an embodiment, the plurality of available measurement sequences includes a first and a second measurement sequences, the first measurement sequence having a shorter overall duration than the second measurement sequence, and/or the first measurement sequence including fewer measurements than the second measurement sequence.

In an embodiment, different measurement sequences mean different physiological measurements and/or different order of physiological measurements.]

In an embodiment, the sensor device comprises at least one of: a weight sensor, an impedance sensor, an ECG sensor, an ESC sensor.

In an embodiment, the predetermined patterns include a spatial distribution of the configuration data.

In an embodiment, the predetermined patterns include a spatial dissymmetry of the configuration data, wherein the spatial dissymmetry being preferably representative of a user stepping on the weighing system with the left foot first or the right foot first.

In an embodiment, the sensor device comprises a plurality of weight sensors (e.g., cells), each weight sensor being configured to measure an associated load, wherein the spatial dissymmetry includes the detection by half or fewer of the weight sensors of a load greater than a first predetermined threshold, the other half or more of the weight sensors detecting a load lower than a second predetermined threshold.

In an embodiment, the predetermined patterns include a temporal distribution of the configuration data.

In an embodiment, the configuration data include: load data, and/or bioimpedance data.

In an embodiment, the control circuitry is configured to receive from an external device customization data comprising mode data to change the available measurement sequences.

In an embodiment, the control circuitry is configured to receive from an external device customization data comprising association data to change the associations between the plurality of predetermined patterns and the plurality of measurement sequences.

In an embodiment, the control circuitry is configured to receive from an external device customization data comprising pattern data to change the predetermined patterns.

In an embodiment, the control circuitry is configured to store a user profile associated to each user of the weighing system, the user profile including notably a height of the user and the sex of the user.

In an embodiment, the control circuitry is configured to select a measurement sequence among the plurality of measurement sequences of the display device based on the configuration data and on a user profile.

In an embodiment, each user profile defines the plurality of available measurement sequences, wherein the user profile further defines the association between the plurality of predetermined patterns and the plurality of available measurement sequences.

In an embodiment, the control circuitry stores a plurality of user profiles, the control circuitry being configured to detect, during a recognition period, a specific user standing on the weighing system and to select the associated user profile.

The disclosure also relates to a method of generating measurement data carried out by a measurement system as described above, the method comprising at least the following steps:
- generating configuration data during a configuration period,
- selecting a measurement sequence among a plurality of available measurement sequences, the selection being based at least on the configuration data,
- generating measurement data during a measurement period according to the selected measurement sequence, the measurement data being representative of a physiological state of the user.

The invention also concerns a computer program comprising software instructions which, when executed by a computer, carry out a method of generating measurement data as described above.

### Brief description of the Drawings

These features and benefits of the invention will appear more clearly upon reading the following description, provided solely as a non-limiting example, and done in reference to the appended drawings, in which:
**[****Fig. 1]** Figure 1 shows a top perspective view along a foot axis of the weighing system according to the invention with a handle in a retracted position in view (a) and in an extended position in view (b),
**[****Fig. 2]** Figure 2 shows a schematical view of the weighing system of Figure 1 communication with external devices,
**[****Fig. 3]** Figure 3 shows a schematic timeline of an operation of the weighing system of Figure 1 according to an embodiment of the description,
**[****Fig. 4]** Figure 4 illustrates an example of a load distribution over time respectively on the left side (top chart) and the right side (bottom chart) of the weighing system, according to an embodiment of the description,
**[****Fig. 5]** Figure 5 illustrates another example of a load distribution over time respectively on the left side (top chart) and the right side (bottom chart) of the weighing system, according to an embodiment of the description,
**[****Fig. 6]** Figure 6 shows two branches of a method of generating measurement data with the weighing system of Figure 1 (schematically represented), according to an aspect of the description, and
**[****Fig. 7]** Figure 7 shows a schematic timeline of an operation of the weighing system of Figure 1 according to another embodiment of the description.

### Detailed description

In the following description, the terms "horizontal" and "vertical" are defined relatively to a weighing system placed on a flat floor. When the weighing system is moved around by a user, the "vertical direction" as defined below may be different from the gravity direction. As illustrated on the Figures, a vertical axis Z, a longitudinal axis X and a transversal axis Y are defined. In normal use, the user stands along the vertical axis Z and the user's feet are positioned along the longitudinal axis Y of the weighing system.

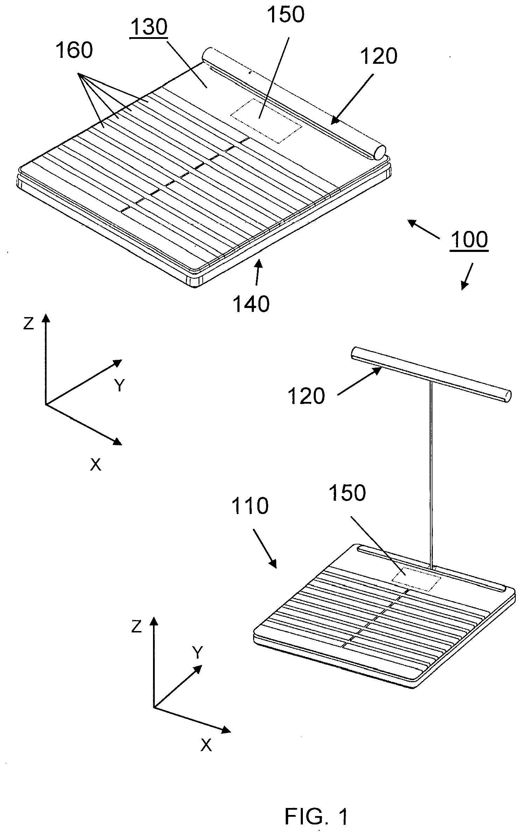

A measurement system 100 is represented on Figure 1. In the illustrated example, this measurement system 100 is an electronic bathroom scale, or personal scale, on which a user can position himself or herself to measure a physiological sign, notably his/her weight. It will thus be referred to as a weighing system 100. In an embodiment, the weighing system 100 may be configured to measure bioimpedance, a body composition (bioimpedance analysis, BIA), an electrocardiogram (ECG), a Pulse Wave Velocity (PWV), a ballistocardiogram (BCG), an electrochemical skin conductance (ESC) analysis, etc.

The weighing system 100 is configured to measure a weight, for example in the range between 5 kg and 300 kg, in particular within the range between 30 kg and 140 kg. Such a device is known as "bathroom scale", but it may obviously be used in a bedroom or in another room of a house.

The weighing system 100 comprises a main body 110, configured to receive the user's feet. The weighing system 100 of Figure 1 may also include a handle 120, to be held by user's hands.

In reference to Figure 1, the handle 110 may be connected to the measurement station by a cable 125. In particular, the cable 125 allows electrical signals to be passed between the handle 120 and the main body 110. In order to have a convenient weighing system 100 without a flying cable, the cable 125 may extend and retract (e.g., wind and unwind) within the main body 110. The cable 125 is flexible to enable an easy wind and unwind. In an extended position, represented on Figure 1 (b), the cable 125 is mechanically recalled towards a retracted position, represented on Figure 1 (a). Thus, the handle is movable at least between the retracted position and the extended position. The handle 120 may include at least one sensor. The handle 120 may be used to perform at least one of the following measurements: ECG (ECG-one lead between the two hands or multiple leads with other limbs), BIA (so-called "segmental"), IPG. The sensors of the handle 120 are selected in particular from: optical sensor for PPG and electrodes.

The main body 110 comprises an upper plate 130, on which the user steps, and a bottom part 140, placed underneath the upper plate 130. In an exemplary embodiment displayed on Figure 1, both the upper plate 130 and the bottom part 140 have a square shape with rounded corners, where both squares are having sides of the same size.

The thickness of the upper plate 130 and the bottom part 140, and the distance between them, are chosen such that the overall thickness of the weighing system 100 along the foot direction Z does not exceed 25 mm. In some embodiments, the thickness of the weighing system 100 may be advantageously reduced to less than 20 mm, and even preferably less than 18 mm. The upper plate 130 and the bottom part 140 form a housing, the housing defining an internal volume.

The main body 110 may include a display device 150 to display information to the user. The display device 150 may be a screen (LCD, LED, electronic, e-ink display). By screen it is meant a dynamic interface capable of displaying alphanumerical or graphical information to the user.

To save energy, the display device 150 is configured to switch from a standby state, in which the display device 150 is switched off, to an awaken state in which the display device 150 is switched on.

The weighing system 100 includes a sensor device, which includes for example a weight sensor or a plurality of weight sensors (also referenced as load sensor) spatially distributed under the upper plate 130 (not visible on Figure 1), a impedance sensor (also called bioimpedance sensor, for which electrodes 160 are visible), an electrocardiogram sensor (for which electrode 160 and the handle 120, with electrodes as well are visible), an electrochemical skin conductance sensor (for which electrode 160 are visible). Document WO2023126220 provides examples of such a weighing system 100.

The weighing system 100 is configured to carry out a sequence of measurements on a user (called a measurement sequence), using the sensor device. For example, a sequence of measurement includes several measurement such as weight, bioimpedance, ECG, PWV, ESC, etc. In particular, as it will be disclosed in more detail later, different sequences of measurements are possible.

The display device 150 may be configured to further display an indicator of the repartition of loads applied on the weighing system 100 to enable a better weighing measurement. The indicator is for example one or several arrows pointing in the direction of a dissymmetry of loads on the upper plate.

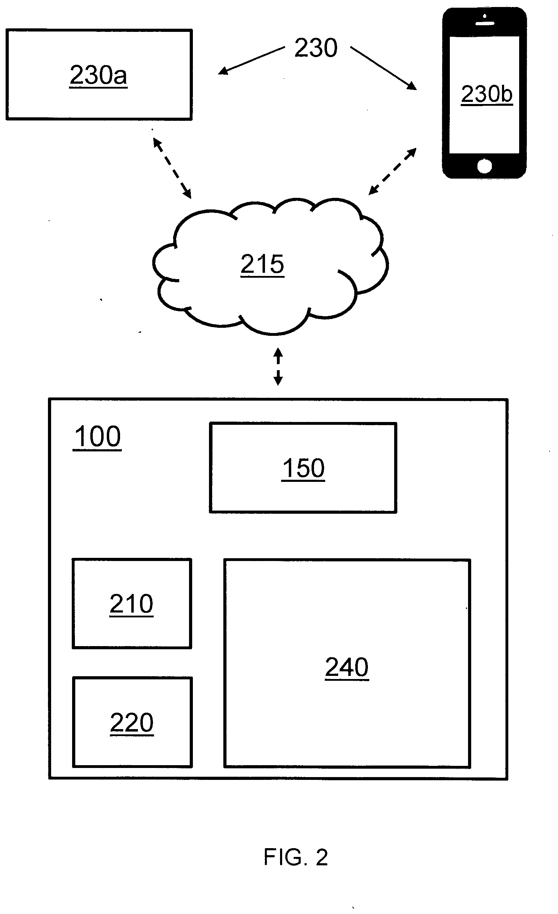

Referring to Figure 2, the weighing system 100 further comprises control circuitry 210 arranged inside the internal volume with a processor and memory and an input/output (I/O) interface, which, among other things, allows the control circuitry to receive and send data from and to a communication network 215. The processor is configured to, among other things, process data obtained by sensors of the weighing system 100. The control circuitry 210 and other electronic components may be mounted on a printed circuit board (PCB), which is attached to the top plate 130, for example.

The control circuitry 210 may store a user profile associated to each user of the weighing system 100. The user profile may include a height of the user and the sex of the user. In addition, to automatically recognize the user, the user profile may include the weight of the user. This weight may be the latest measurement or an average of the latest measurements. Those data of the user profile are used to compute the body mass index (BMI) and the body composition of the user. The weighing system 100 further comprises a battery 220 (e.g., rechargeable) arranged inside the internal volume configured for supplying power to the various components of the weighing system 100.

The weighing system 100, and in particular the input/output (I/O) interface, may communicate with at least an external device 230 via the communication network 215, which may include a wireless network (in particular a network compatible with at least one of the following communication protocols: Bluetooth, Wi-Fi, cellular, etc.). The external devices 230 may include a server 230a and/or a mobile terminal 230b (smartphone, etc.). The weighing system 100 may communicate with the server 230a and/or the mobile terminal 230b. In one implementation, the weighing system 100 may communicate directly with the mobile terminal 230b, for example via Bluetooth or Bluetooth Low Emission (BLE). This communication may be implemented at the installation of the weighing system 100, in particular to pair it with the mobile terminal 230b and/or to configure a connection to the server 230a that does not transit through the mobile terminal 230b and/or as a backup for a failed communication with the server 230a. In one embodiment, the weighing system 100 may communicate directly with the server 230a, without transiting through the mobile terminal 230b. This communication allows the user to use the weighing system 100 even without having the mobile terminal 230b nearby.

The control circuity 210 stores the plurality of measurement sequences. This allows the weighing system to operate the several embodiments of the description without communicating with an external device 230.

The weighting system 100 further comprises the sensor device, referenced as 240 and described above. The sensor device 240 is configured to generate several data, as it will be further explained below, such as configuration data and measurement data. By data generation, it is understood the measurement of a physical property relative to the user, in particular the measurement of a physical property over space and/or over time.

As mentioned before, in an embodiment, the sensor device 240 comprises weight sensors. The weight sensors are positioned between the upper plate 130 and the bottom part 140 (in so-called "sandwich" architecture) or, in a variant, between the upper plate 130 and feet, not shown, (in a so-called "foot" architecture). The weight sensors may be load cells (typically four) that provide a weight, and thus a mass of a user.

The control circuitry 210 is configured to switch the display device 150 into a standby state when the sensor device 240 detects a load below a predetermined standby threshold for a predetermined standby duration, for example under 2 kg during 2 s. The control circuitry 210 is configured to awaken the display device 150 by switching it to the awaken state when the sensor device 240 detects a load greater than a predetermined awakening threshold for a predetermined awakening duration, for example over 2 kg during more than 1 s.

The sensor device 240 is configured to generate measurement data during a measurement period and configuration data during a configuration period.

Measurement data are data generated by an interaction of the user with the weighing system 100, in particular with the sensor device 240, in order to obtain a physiological state about his or her body (e.g., weight, fat mass, nerve health activity, BCG, ECG, PWV, etc.). During the measurement period, the user usually stands still on the weighing system 100, so that the sensor device 240 may correctly generate the measurement data. The measurement data is configured to be displayed, during a measurement display period, by the display device 150. The measurement display period may follow the measurement period or may take place in parallel of the measurement period. In other words, during the measurement period, the user usually needs to be still and passive for the measurement data to be properly generated by the sensor device 240.

Configuration data are data generated by an interaction of the user with the weighing system 100, particular with the sensor device 240, in order to select a measurement sequence. The choice is carried out amongst a plurality of available measurement sequences that are stored in the control circuitry 210. During the configuration period, the user's behavior on the weighing system 100 affects the configuration data and therefore enables him or her to choose a measurement sequence to be carried out. In other words, during the configuration data, the user needs to be active for the configuration data to be properly generated by the sensor device 240. Using the configuration data, the control circuitry 210 selects a measurement sequence.

Different sensors of the sensor device 240 may be used for the configuration data and the measurement data. Typically, in an embodiment, the configuration data are generated using the weight sensor of the sensor device 240 and those configuration data trigger a measurement sequence which involves (or does not involve) a measurement using another sensor of the sensor device 240.

### The measurement sequences

The selection of the measurement sequence is based at least on the configuration data. The sensor device 240 is then configured to generate measurement data during the measurement period according to the selected measurement sequence.

The measurement sequence includes at least one physiological measurement of the user by the sensor device 240 to generate measurement data. The measurement data is representative of a physiological state of the user. Each measurement sequence may comprise one or a plurality of physiological measurements (involving each one or more sensors of the sensor device 240). In particular, each measurement sequence may comprise between one and ten physiological measurements. For measurement sequences involving more than one measurements, the measurement sequence is also defined by the order of the measurements.

At least one physiological measurement comprises at least one of: weight or mass measurement including ballistocardiogram (BCG) measurement; electrocardiogram (ECG) measurement; bioimpedance measurement including impedance-plethysmogram (IPG) measurement, impedance-cardiogram (ICG) measurement and/or bioimpedance analysis (BIA) measurement; photoplethysmogram (PPG) measurement;, electrochemical skin conductance analysis ("ESC analysis") measurement, heart rate ("HR") measurement, pulse wave velocity ("PWV") measurement, and/or heart failure measurement. These measurements are carried out using the sensor device 240.

Each physiological measurement is associated to a physiological sign and at least a measurement parameter. The physiological measurement comprises the measurement of the physiological sign according to the at least one measurement parameter.

A physiological sign is a physiologic property over space and/or over time reflecting essential body functions, and therefore reflecting the user general physiologic state as well as specific disease states. Each physiological sign may be for example heartbeat, breathing rate, temperature, blood pressure, blood oxygen saturation, weight, body fat mass percentage, pulse wave velocity, electrochemical skin conductance, etc.

A measurement parameter is an option, a factor, a limit, a constant, etc. which determines (e.g., establishes or limits) how the physiological measurement is carried out. A measurement parameter is for example a measurement duration, a choice of measurement algorithm (e.g., different body parameters depending on the sex of the user for BIA), a choice of measurement sensor, etc.

For example, an ECG measurement is a physiological measurement enabling to measure and to monitor the regularity of a user heartbeat. The measurement parameters may be a measurement duration (for example 30 seconds or 1 minute) or the choice of an atrial fibrillation detection algorithm or a choice in a number of leads (1 lead, 6 leads, etc.). As a consequence, a 30 second lasting ECG measurement is considered as being a different physiological measurement than a 1 minute lasting ECG measurement.

As another example, a BIA measurement is a physiological measurement enabling to measure a body fat mass. The measurement parameter may be the choice of a calculation algorithm. As a consequence, a BIA measurement with a conventional algorithm is considered as being a different physiological measurement than a BIA measurement with an "athlete mode" algorithm.

In an embodiment, all the available measurement sequences are different from one another. Different measurement sequences mean different physiological measurements (e.g., a first sequence being weight, BIA, and ECG while a second sequence is weight and BIA) and/or different order of physiological measurements (e.g., a first sequence being weight, BIA and ECG, while a second sequence is weight, ECG and BIA).

In an embodiment, at least an available measurement sequence comprises strictly fewer physiological measurements than the other measurement sequences. For example, one of the available measurement sequence may comprise only a weight measurement while other measurement sequences may comprise two or more physiological measurements. Therefore, the user may choose each time he/she steps on the scale, if he/she wants only a weight measurement or a more complete heath scan.

In a variant or in complement, at least an available measurement sequence has an overall duration shorter than another one (e.g., without the ECG). In other words, at least an available measurement sequence lasts temporarily less than the other measurement sequences. For example, one of the measurement sequence may comprise only quick measurements while other measurement sequences may comprise more longer physiological measurements. For example, the user may choose at each time he/she steps on the scale, if he/she wants to make a 30 second or 1 minute long ECG.

In a variant or in complement, two measurement sequences may comprise the same physiological measurements but in a different order.

In a variant or in complement, two measurement sequences may comprise different physiological measurements. For example, the user may choose to alternate two different measurements sequences to obtain a complete health check while reducing the duration of each measurement sequence.

### The configuration period

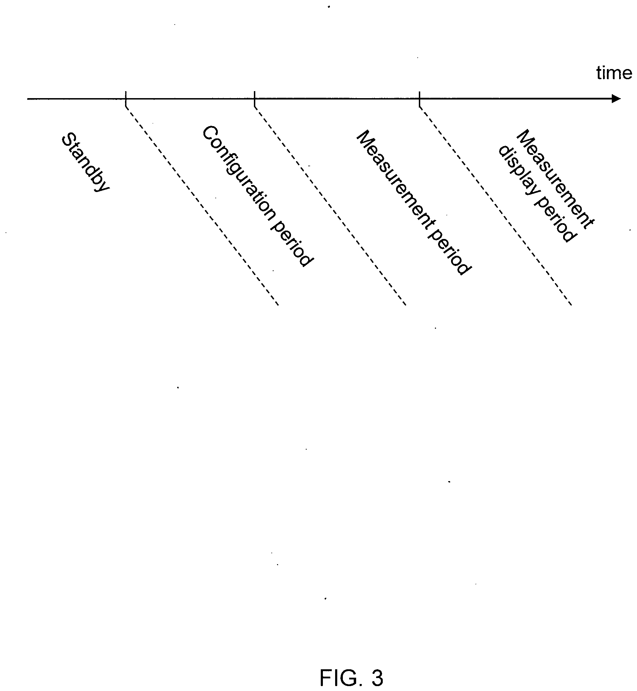

The operation of the weighing system 100 during the configuration period will now be described in reference to Figures 3 to 5. The configuration data are generated using at least one sensor of the sensor device 240.

In an embodiment, the configuration data are load data and the sensor device 240 includes weight sensors, such as load cells, to generate load data. In another example of the pattern embodiment, the configuration data are impedance data and the sensor device 240 includes impedance sensors, such as electrodes (and the associated electronics: voltage or current generators, voltmeter, etc.).

Referring to Figure 3, the configuration period is advantageously carried out following each awakening of the display device 150 from the standby state. The configuration period is a period lasting for example less than 5 s, notably less than 2s.

As mentioned above, the selection of the measurement sequence is based at least on the configuration data. The sensor device 240 is then configured to generate measurement data during the measurement period according to the selected measurement sequence.

In particular, the selection of the measurement sequence is based at least on an identification in the configuration data of a pattern amongst a plurality of predetermined patterns associated to the plurality of available measurement sequences. By "pattern", it is understood a particular spatial and/or temporal structure of the configuration data, that may be repeatable. One of the measurement sequences (called the default measurement sequence) may be associated to an absence of identification of a predetermined pattern.

By spatial, it is meant depending on the position or the movement of the user on the upper plate 130 of the weighing system 100; of course, such position may evolve over time. By temporal, it is meant of the movement of the user on the upper plate 130. A pattern thus provides a simplified representation of a specific user behavior, such as a stepping on the weighing system 100, as it will be further explained below.

The predetermined patterns and their association with their respective available measurement sequences are stored in the control circuitry 210. As mentioned previously, the plurality of available measurement sequences is also stored in the control circuitry 210. The predetermined patterns and the plurality of measurement sequences may be stored permanently in the control circuitry 210.

For example, the control circuitry 210 is configured to compare the configuration data to each of the predetermined patterns.

The comparison may comprise determining a distance (according to metric) between the configuration data and each of the predetermined patterns. The control circuitry 210 may identify the predetermined pattern with the lowest distance and selects the measurement sequence associated with the identified predetermined pattern.

The comparison may comprise comparing the configuration data with thresholds forming the predetermined patterns. The control circuitry 210 may identify the predetermined pattern based on the threshold comparison and select the measurement sequence associated with the identified predetermined pattern.

The measurement period follows the configuration period, as illustrated on Figure 3. The measurement data may be weight data of the user. The weight measurement is then carried out by the load cells.

In a variant or in complement, the measurement data may be: body composition data, bioimpedance data, ECG data, PWV data, nerve health data.

### Spatial distribution for the predetermined patterns

The predetermined patterns may cover a spatial distribution of the configuration data on the upper plate 130. For example, the predetermined patterns may be configured to distinguish a user stepping with the left foot or the right foot first on the upper plate 130 of the weighing system 100 during the configuration period (called "left foot first" pattern or "right foot first" pattern in the rest of the description). The control circuitry 210 identifies in the measurement data a pattern amongst the plurality of predetermined patterns and then selects the measurement sequence associated to the identified predetermined pattern.

In an implementation, the predetermined patterns cover a spatial dissymmetry of the configuration data during the configuration period.

### Predetermined load pattern

In an example of this embodiment, the predetermined patterns may be predetermined load patterns distributed over space. In particular, a load pattern is here a spatial distribution of the load between the plurality of weight sensors of the sensor device 240. As each weight sensor is able to measure a load, the load pattern is representative of a predetermined load repartition between the weight sensors. The load pattern notably includes a spatial dissymmetry of the load between the plurality of weight sensors of the sensor device 240 during the configuration period. For example, the dissymmetry is the detection by half of the weight sensors of a load greater than a first predetermined threshold, the other half of the weight sensors detecting a load lower than a second predetermined threshold (half for an even numbers of weight sensors, otherwise the number rounded up and rounded down).

Concretely, the dissymmetry may be representative of a user stepping on the weighing system 100 with the left foot (or alternatively with the right foot). In this case, the weight sensors located on the left side (or alternatively on the right side) detects a load due to the user stepping on and the weight sensors located on the other side do not detect any load. The control circuitry 210 is configured to detect this dissymmetry, to associate it to a predetermined pattern and to select the associated measurement sequence. For example, if the control circuitry 210 detects that the user stepped on the weighing system 100 with the right foot, the control circuitry 210 selects a first measurement sequence. If the control circuitry 210 detects that the user stepped on the weighing system 100 with the left foot, the control circuitry 210 selects another measurement sequence.

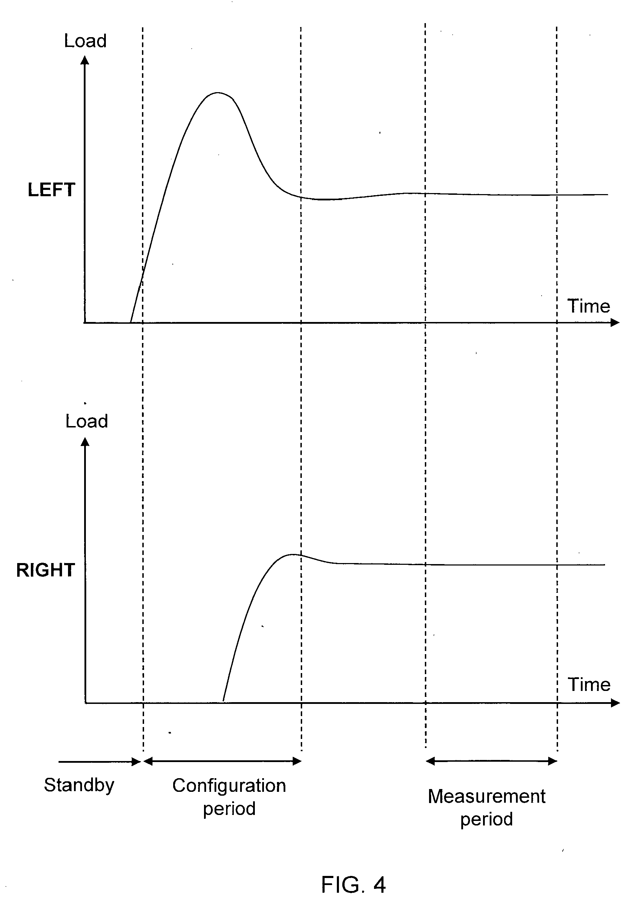

Figure 4 illustrates the load versus time evolution on respectively the left side and the right side of the sensor device 240. As visible on the two graphs of Figure 4, the device display 150 is in standby mode when no load is applied on the weighing system 100. Then, when the user steps with his or her left foot first on the weighing system 100, the left side of the sensor device 240 detects the applied load and the display device 150 is awaken. Then, during the configuration period, the control circuitry 210 detects a difference of loads between the left and the right sides of the upper plate 130. In particular, the control circuitry 210 detects a difference of load greater than a predetermined threshold, for example 30 kg, during a predetermined period, for example 2s. The control circuitry 210 detects therefore the pattern associated to a user stepping on the weighing system 100 with the left foot and selects the associated measurement sequence. Then, the sensor device 240 applies the selected measurement sequence.

### Predetermined impedance pattern

In another example of this embodiment, the predetermined patterns are impedance patterns. In particular, an impedance pattern is here a spatial distribution of the impedance between the impedance sensors. As each impedance sensor is able to measure an impedance, the impedance pattern is representative of a predetermined impedance repartition between the impedance sensors. The load pattern notably includes a spatial dissymmetry of the impedance between the plurality of impedance sensors during the configuration period. For example, the dissymmetry is the detection by half of the impedance sensors of an impedance lower than a first predetermined threshold, the other half of the impedance sensor detecting an impedance higher than a second predetermined threshold.

Concretely, the impedance sensors include two impedance sensors, one for each foot (as implemented on any impedance scale). The dissymmetry may be representative of a user stepping on the weighing system 100 with the left foot (or alternatively with the right foot). In this case, the impedance sensor located on the left side (or alternatively on the right side) detects an impedance due to the user stepping on and the impedance sensor located on the other side do not detect any impedance. The control circuitry 210 is configured to detect this dissymmetry, to associate it to a predetermined pattern and to select the associated measurement sequence. For example, if the control circuitry 210 detects that the user stepped on the weighing system 100 with the right foot, the control circuitry 210 selects a first measurement sequence. If the control circuitry 210 detects that the user stepped on the weighing system 100 with the left foot, the control circuitry 210 selects another measurement sequence.

### Temporal distribution for the predetermined patterns

The predetermined patterns may cover a temporal distribution of the configuration data on the upper plate 130. For example, the predetermined pattern may be configured to distinguish a load variation in time (for example a tapping pattern or a knee flexion on the upper plate).

In an implementation, the predetermined patterns may be predetermined load patterns distributed over time. The predetermined patterns here may be representative of an evolution of the load during time regardless of the spatial distribution of the load (therefore not a right/left dissymmetry of loads as above).

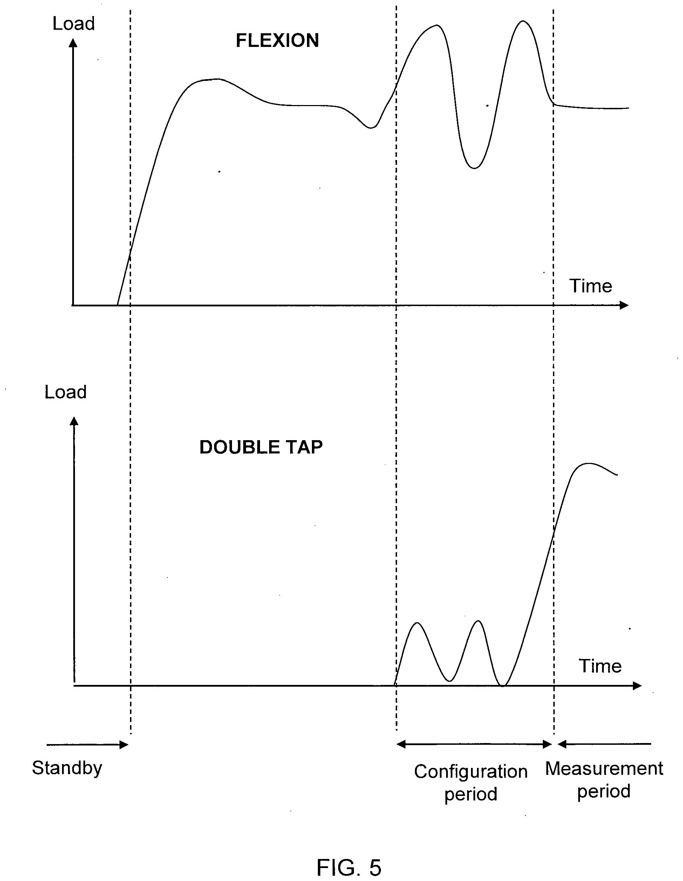

For example, as illustrated on FIG. 5 ("FLEXION") a predetermined pattern may be a flexion (two flexions on FIG. 5) with knees done by the user on the upper plate 130 after stepping on the weighing system 100. The predetermined patterns could include any of one or two flexions. As visible on Figure 5, the device display 150 is in standby mode when no load is applied on the weighing system 100. Then, when the user steps with his or her left foot first on the weighing system 100, the weighing system 100 detects the applied load and the display device 150 is awaken. Then, during the configuration period, the control circuitry 210 detects a double flexion made by the user. In particular, the control circuitry 210 may detect two peaks of loads by comparison with a predetermined threshold (e.g., above a certain variation of the weigh). The control circuitry 210 detects the predetermined pattern associated to a user double flexing on the weighing system 100 and selects the associated measurement sequence. Then, the sensor device 240 carries out the selected measurement sequence.

In a variant, as illustrated on FIG. 5 ("DOUBLE TAP"), a predetermined pattern may be a tap (double tap on FIG. 5) with a foot done by the user on the upper plate 130 before stepping on the weighing system 100: during the configuration period, the control circuitry 210 detects a double tap made by the user. In particular, the control circuitry 210 may detect two small peaks of loads by comparison with threshold (for example between two predetermined thresholds). The control circuitry 210 detects the predetermined pattern associated to a user double taping on the weighing system 100 and selects the associated measurement sequence. If not taping is detected, then no predetermined pattern is identified and therefore the default measurement sequence may be selected (e.g., the only weight measurement sequence). The double tap may be done by the user indifferently on the right side, the left side or on the center of the upper plate 130.

For example, instead of a double tap, the predetermined patterns may include different durations of a single tap, or different durations for any tap of a double tap.

### Temporal and spatial distribution for the predetermined patterns

The predetermined patterns may cover a temporal and spatial distribution of the configuration data on the upper plate 130. This allows for more predetermined patterns, as for example they may include the following: left foot first, right foot first, left double tap, right double tap, left tap then right tap, right tap then left tap, etc.

### DESCRIPTION OF THE OPERATION OF THE WEIGHING SYSTEM

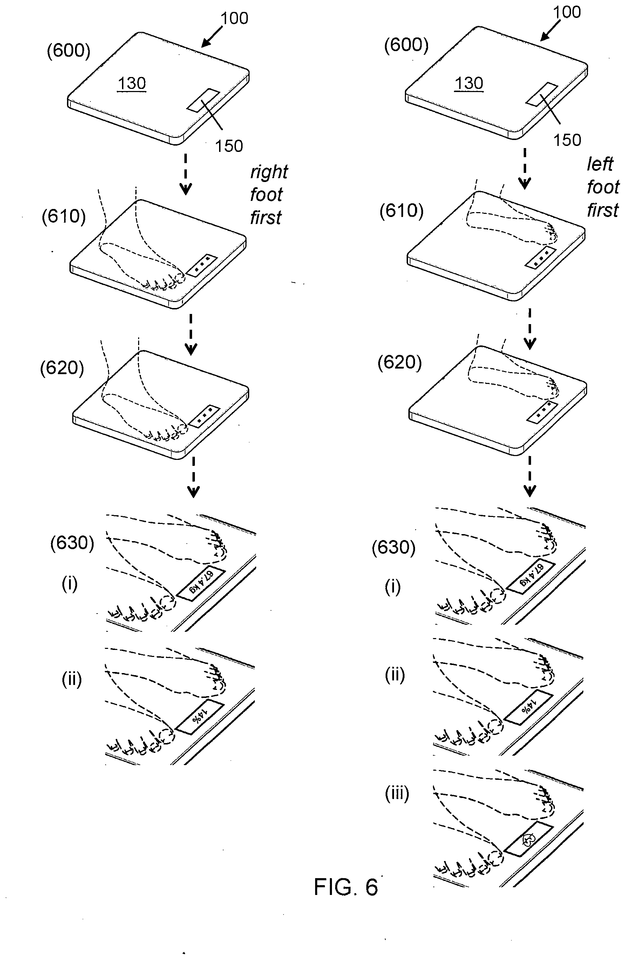

A method of generating measurement data with the weighing system 100 (schematically represented as no electrodes are visible for example) according to the pattern embodiment will now be described, in reference to Figure 6, using an illustrative example. In the illustrative example, the configuration data are load configuration data and the predetermined patterns correspond to a "left foot first" pattern and a "right foot first" pattern.

The "right foot first" pattern is associated with a measurement sequence comprising only a weight measurement and a bioimpedance measurement (e.g., body fat measurement). The "left foot first" pattern is associated with a measurement sequence comprising a weight measurement, a bioimpedance measurement, and an ECG measurement.

Initially, at step 600, the display device 150 is in the standby mode and nothing is displayed by the display device 150, as illustrated on Figure 6. More generally, the weighing system is in a standby mode.

Then, at step 610, the sensor device 240 generates the configuration data during the configuration period as the user starts interacting with the weighing system. In particular, the user steps on the weighing system 100 with a chosen foot - the right foot in the following example.

In the left foot first branch of Figure 6, because the user steps on the upper plate 130 with the left foot first, a "left foot first pattern" is identified by the control circuitry 210 and the measurement sequence associated to said pattern would have been selected by the control circuitry 210. Therefore, at step 620, the control circuitry 210 identifies a load pattern (here a load dissymmetry called "left foot first" pattern) in the configuration data and selects the measurement sequence associated to the identified load pattern. In this example, the associated measurement sequence is thus the measurement sequence with only a weight measurement.

In the right foot first branch of Figure 6, because the user steps on the upper plate 130 with the right foot first, the sensor device generates at step 810 configuration data during the configuration period. Therefore, at step 620, the control circuitry 210 identifies a load pattern (here a load dissymmetry called "right foot first" pattern) in the configuration data and selects the measurement sequence associated to the identified load pattern. In this example, the associated measurement sequence is thus the measurement sequence with a weight measurement, a body fat measurement and an ECG measurement.

After the configuration period, the user brings his or her other foot on the upper plate 130, the user has his or her two feet on the weighing system 100 and the sensor device 240 generates, at step 630, the measurement data during the measurement period according to the selected measurement sequence.

In particular, in the example of Figure 6, in the right foot first branch, the sensor device 240 measures successively the weight of the user at 630(i), and then the bioimpedance of the user at 630(ii). In the left foot first branch, the sensor device 240 measures successively the weight of the user at 630(i), the bioimpedance of the user at 630(ii), and then an ECG of the user at 630(iii). Given that an ECG usually takes at least 30s, the right foot first branch has an overall duration that is at least 30s shorter than the left foot first branch.

The display device 150 may display the results of the measurements taken.

During the weight measurement, the display device 150 may display an indicator of the dissymmetry of the loads applied on the upper plate 130 and thus the lad sensor to enable a better weigh measurement. The indicator is here two arrows pointing in the direction of the user indicating that the user needs to lean forward.

### CUSTOMIZATION OF THE MEASUREMENT SEQUENCE

A customization feature of the measurement sequences with now be described. This customization applies to all the embodiments of the description.

Referring again to Figure 2, the control circuitry 210 is configured to communicate with the external device 230. In this embodiment, the external device 230 is preferably a personal external device 230 that the users may easily have (e.g., a smartphone, a tablet, or even a personal computer such as a laptop).

The control circuitry 210 may be configured to receive from the external device 230 customization data to personalize the experience with the weighing system.

The customization data may include any of: sequence data to modify the available measurement sequences, pattern data to change the predetermined patterns, association data to change the association between the predetermined patterns and the available measurement sequences, activation data to selectively activate or deactivate the configuration period and to select a default measurement sequence when the configuration period is deactivated.

### Mode data

For example, the user may choose on the external device 230b (on an app for example) that the available measurement sequences he or she wishes to have on the weighing system 100. This choice generates customization data with mode data and the external device 230b is configured to send the customization data to the control circuitry 210 of the weighing system 100. The control circuitry 210 is configured to receive the customization data and to modify the available measurement sequences. For example, a user may want a specific physiological measurement to become present in each sequence and another specific physiological measurement to become optional.

### Pattern data

For example, the user may choose on the external device 230b (on an app for example) the predetermined patterns that he or she wants to execute on the weighing system 100, for example among a list of predetermined patterns. This choice generates customization data with pattern data and the external device 230b is configured to send the customization data to the control circuitry 210 of the weighing system 100. The control circuitry 210 is configured to receive the customization data and to change, using those customization data, the predetermined patterns. For example, the user may want to predetermined patterns to be a temporal distribution of the configuration data rather than a spatial distribution of the configuration data.

### Association data

For example, the user may choose on the external device 203b (on an app for example) the predetermined pattern he or she wishes to associate to each measurement sequence. This generates customization data with association data and the mobile terminal 230b is configured to send the customization data to the control circuitry 210 of the weighing system 100. The control circuitry 210 is configured to receive the customization data and to change, using those customization data, the association between predetermined patterns and measurement sequences. For example, if initially stepping on the weighing system 100 with the left foot is associated with only measuring weight and stepping with the right foot is associated with measuring weight and body composition, the user may want to swap these two predetermined patterns. The user may therefore change the settings in an app of his or her external terminal 230b so that stepping on the weighing system 100 with the left foot is now associated to measuring weight and body composition and stepping with the right foot is associated to measuring only weight.

### Activation data

The control circuitry 210 may be configured to receive from the external device 230 activation data to selectively activate or deactivate the configuration period and to select a default measurement sequence when the configuration period is deactivated. In other words, the user may want to temporarily disable the pattern recognition and may want to always use the same measurement sequence. For example, the user may have little time available and want to record only his or her weight regularly. The user may therefore deactivate the configuration period and choose the only weighing measurement sequence to be applied every time. After a few weeks, the user may have more time and may want to carry out more physiological measurement. The user may then reactivate the configuration period.

### CUSTOMIZATION BASED ON THE USER PROFILE

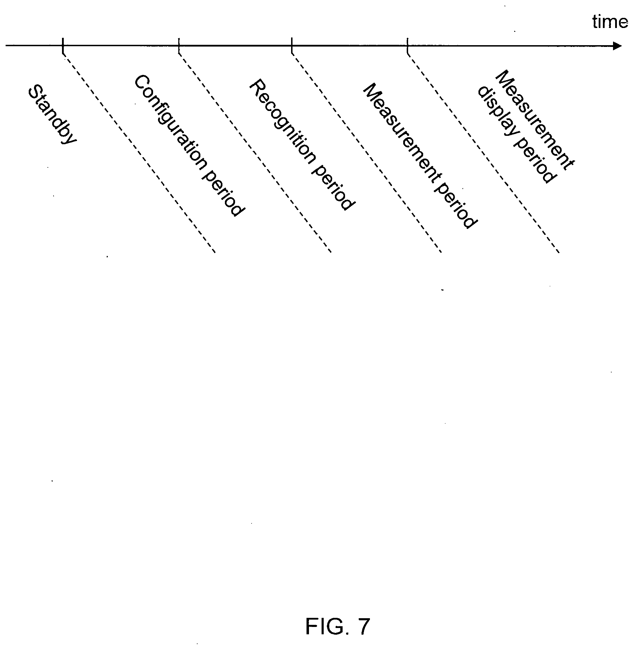

This time, in relation to Figure 7, the experience may vary based on the user recognized the weighing system 100.

The control circuitry 210 may be configured to receive from the external device 230 and to store at least a user profile associated to a user of the weighing system 100. In particular, a plurality of user profiles may be stored in the control circuitry 210. As indicated above, the user profile typically includes a heigh of the user, the sex of the user and, potentially, the age of the user.

In an implementation, the customization data are linked to a user profile. Therefore, in the weighing system 100, the available measurement sequences and, in the relevant embodiment, their association with a predetermined pattern depends on the user profile.

The control circuitry 210 is configured to detect a specific user standing on the weighing system 100 among the plurality of user profiles during a recognition period and to select his or her user profile. As illustrated on Figure 10, the recognition period follows the configuration period. The recognition period may occur before the measurement period as illustrated. In a variant, the recognition period may take place in parallel of the measurement period. The control circuitry 210 may recognize the specific user based on a measured weight and a weight linked to a user profile (e.g., by comparing the measured weigh and said linked weigh of the user profiles). In this embodiment, each measurement sequence comprises at least a weight measurement. Following the recognition, the control circuitry 210 applies the rest of the selected measurement sequence.

The control circuitry 210 is configured to select a measurement sequence based on the configuration data and based on a user profile. In other words, the selection of a measurement sequence by the control circuitry 210 may depend on the pattern the user carries out while stepping on the weighing system 100 and on his/her user profile. For example, user A may want the only weight measurement sequence when stepping with the right foot first and user B may want this only weight measurement sequence when stepping with the left foot first. Thanks to the recognition period, the control circuitry is able to recognize the user and to select his or her user profile and to apply the appropriate measurement sequence associated with the appropriate predetermined pattern.

## Claims

1. A measurement system (100) comprising:
- a sensor device (240) configured to generate configuration data during a configuration period and measurement data during a measurement period, the measurement data being representative of a physiological state of the user,
- control circuitry (210) configured to select a measurement sequence among a plurality of available measurement sequences, wherein each measurement sequence includes at least one physiological measurement of the user to generate measurement data, the selection being based at least on the configuration data,
wherein:
- the selection is based at least on an identification in the configuration data of a pattern amongst a plurality of predetermined patterns associated to the plurality of available measurement sequences,
- the sensor device (240) is further configured to generate the measurement data according to the selected measurement sequence.

2. The measurement system (100) according to any of the preceding claims, wherein each physiological measurement is associated to a physiological sign and at least a measurement parameter, said physiological measurement comprising the measurement of the physiological sign according to the at least one measurement parameter.

3. The measurement system (100) according to claim 1 or 2, wherein the at least one physiological measurement comprises at least one of:
- weight or mass measurement,
- electrocardiogram (ECG) measurement,
- impedance measurement including impedance-plethysmogram (IPG) measurement, impedance-cardiogram (ICG) measurement and/or bioimpedance analysis (BIA) measurement,
- photoplethysmogram (PPG) measurement,
- ballistocardiogram (BCG) measurement,
- electrochemical skin conductance analysis ("ESC analysis") measurement,
- heart rate ("HR") measurement,
- pulse wave velocity ("PWV") measurement, and/or
- heart failure measurement.

4. The measurement system (100) according to any of the preceding claims, wherein all the available measurement sequences are different from one another.

5. The measurement system (100) according to any of the preceding claims, wherein the plurality of available measurement sequences includes a first and a second measurement sequences, the first measurement sequence having a shorter overall duration than the second measurement sequence, and/or the first measurement sequence including fewer measurements than the second measurement sequence.

6. The measurement system (100) according to any of the preceding claims, wherein the predetermined patterns include a spatial distribution of the configuration data.

7. The measurement system (100) according to any of the preceding claims, wherein the predetermined patterns include a spatial dissymmetry of the configuration data, wherein the spatial dissymmetry being preferably representative of a user stepping on the weighing system with the left foot first or the right foot first.

8. The measurement system (100) according to claim 7, wherein the sensor device (240) comprises a plurality of weight sensors (e.g., cells), each weight sensor being configured to measure an associated load, wherein the spatial dissymmetry includes the detection by half or fewer of the weight sensors of a load greater than a first predetermined threshold, the other half or more of the weight sensors detecting a load lower than a second predetermined threshold.

9. The measurement system (100) according to any of the preceding claims, wherein the predetermined patterns include a temporal distribution of the configuration data.

10. The measurement system (100) according to any of the preceding claims, wherein the configuration data include:
- load data, and/or
- bioimpedance data.

11. The measurement system (100) according to any of the preceding claims, wherein the control circuitry (210) is configured to receive from an external device (230) customization data comprising at least one of:
- mode data to change the available measurement sequences,
- association data to change the associations between the plurality of predetermined patterns and the plurality of measurement sequences, and/or
- pattern data to change the predetermined patterns.

12. The measurement system (100) according to any of the preceding claims, wherein the control circuitry (210) is configured to store a user profile associated to each user of the weighing system (100), the user profile including notably a height of the user and the sex of the user.

13. The measurement system (100) according to claim 12, wherein the control circuitry (210) is configured to select a measurement sequence among the plurality of measurement sequences of the display device (150) based on the configuration data and on a user profile.

14. The measurement system (100) according to claim 12 or 13, wherein each user profile defines the plurality of available measurement sequences, wherein the user profile further defines the association between the plurality of predetermined patterns and the plurality of available measurement sequences.

15. A method of generating measurement data carried out by a weighing measurement (100) according to any of the preceding claims, the method comprising at least the following steps:
- generating configuration data during a configuration period,
- selecting a measurement sequence among a plurality of available measurement sequences, the selection being based at least on the configuration data,
- generating measurement data during a measurement period according to the selected measurement sequence, the measurement data being representative of a physiological state of the user.
